# EUROPEAN PATENT APPLICATION

(11) **EP 1 767 651 A1**
(43) Date of publication of application: **28.03.2007**
(21) Application number: 05020945.1
(22) Date of filing: 26.09.2005
(51) Int. Cl.: C12Q 1/68

(54) **oriC specific nucleic acid sequences and use thereof**

(71) Applicant: Ludwig-Maximilians-Universität München, 80359 München (DE)
(72) Inventor: Roggenkamp, Andreas, 93036 Passau (DE)
(74) Representative: Bohmann, Armin K.

(57) **Abstract**

The present invention is related to a combination of at least a first nucleic acid molecule and at least a second nucleic acid molecule for the detection of and/or discrimination between bacteria of the group of coliforms and bacteria of the *Escherichia coli* genogroup, whereby the first nucleic acid and the second nucleic acid differ in their nucleic acid sequence and comprise fragments of the origin of replication (*oriC*) locus sequence of said bacteria.

SEQUENCE LISTING

<110> Ludwig-Maximilians-Universität München

<120> oriC specific nucleic acid sequences and use thereof

<130> U 10015 EP

<160> 49

<170> PatentIn version 3.1

<210> 1
<211> 25
<212> DNA
<213> Artificial

<220>
<221> misc_feature
<223> primer

<400> 1
caaaaggatc ckgataaaac atggt 25

<210> 2
<211> 20
<212> DNA
<213> Artificial

<220>
<221> misc_feature
<223> primer

<400> 2
cgggccgtgg attctactca 20

<210> 3
<211> 21
<212> DNA
<213> Artificial

<220>
<221> misc_feature
<223> primer

<400> 3
catgagattc cggargatcc g 21

<210> 4
<211> 20
<212> DNA
<213> Artificial

<220>
<221> misc_feature
<223> primer

<400>  4
cactgccctg tggataacaa                                                    20


<210>  5
<211>  21
<212>  DNA
<213>  Artificial

<220>
<221>  misc_feature
<223>  primer


<400>  5
tatacagatc gtgcgatcta c                                                  21


<210>  6
<211>  290
<212>  DNA
<213>  E. fergusonii

<220>
<221>  misc_feature
<223>  partial oriC locus


<400>  6
ggatcctggg tattaaaaag aagatctatt tatttagaga tctgttctat tgtgatctct      60

tattaggatc gcactgccct gtggataaca aggatccggc ttttaagatc aacaacctgg     120

aaaggatcat taactgtgaa tgatcggtga tcctggaccg tataagctgg gatcagaatg     180

aggggttata cacaactcaa aaactgaaca acggttgttc tttggataac taccggttga     240

tccaagcttc ctgacagagt tatccacagt agatcgcacg atctgtatac                290


<210>  7
<211>  290
<212>  DNA
<213>  E. coli

<220>
<221>  misc_feature
<223>  partial oriC locus


<400>  7
ggatcctggg tattaaaaag aagatctatt tatttagaga tctgttctat tgtgatctct      60

tattaggatc gcactgccct gtggataaca aggatccggc ttttaagatc aacaacctgg     120

aaaggatcat taactgtgaa tgatcggtga tcctggaccg tataagctgg gatcagaatg     180

aggggttata cacaactcaa aaactgaaca acagttgttc tttggataac taccggttga     240

tccaagcttc ctgacagagt tatccacagt agatcgcacg atctgtatac                290

<210> 8
<211> 290
<212> DNA
<213> Shigella boydii

<220>
<221> misc_feature
<223> partial oriC locus


<400> 8
ggatcctggg tattaaaaag aagatctatt tatttagaga tctgttctat tgtgatctct 60

tattaggatc gcactgccct gtggataaca aggatccggc ttttaagatc aacaacctgg 120

aaaggatcat taactgtgaa tgatcggtga tcctggaccg tataagctgg gatcagaatg 180

aggggttata cacaactcaa aaactgaaca accgttgttc cttggataac taccggttga 240

tccaagcttc ctgacagagt tatccacagt agatcgcacg atctgtatac 290


<210> 9
<211> 290
<212> DNA
<213> Shigella flexneri

<220>
<221> misc_feature
<223> partial oriC locus


<400> 9
ggatcctggg tattaaaaag aagatctatt tatttagaga tctgttctat tgtgatctct 60

tattaggatc gcactgccct gtggataaca aggatccgat ttttaagatc aacaacctgg 120

aaaggatcat taactgtgaa tgatcggtga tcctggaccg tataagctgg gatcagaatg 180

aggggttata cacaactcaa aaactgaaca acggttgttc tttggatagc taccggttga 240

tccaagcttc ctgacagagt tatccacagt agatcgcacg atctgtatac 290


<210> 10
<211> 290
<212> DNA
<213> Shigella dysenteriae

<220>
<221> misc_feature
<223> partial oriC locus


<400> 10
ggatcctggg tattaaaaag aagatctatt tatttagaga tctgttctat tgtgatctct 60

tattaggatc gcactgccct gtggataaca aggatccggc ttttaagatc aacaaccttg 120

aaaggatcat taactgtgaa tgatcggtga tcctggaccg tataagctgg gatcagaatg 180

aggggttata cacaactcaa aaactgaaca accgttgttc cttggataac taccggttga 240

tccaagcttc ctgacagagt tatccacagt agatcgcacg atctgtatac 290

<210> 11
<211> 290
<212> DNA
<213> Citrobacter amalonaticus

<220>
<221> misc_feature
<223> partial oriC locus

<400> 11
ggatcctggc tattaaaaag aagatctttt tatttagaga tctgttctat tgtgatctct 60

tattaggatc gccattggct gtggataagc cggatcccgg tattaagatc aataccttgg 120

taaggatcat tagctgtgaa tgatcggtga tcctggtccg tataagctgg gatcagaatg 180

aagggttata cacaactcaa aaactgaaca atggttattc tttggataaa tactggttga 240

tccaaggttc ctgacagagt tatccacaca cgatcgcacg atctttacac 290

<210> 12
<211> 290
<212> DNA
<213> Citrobacter amalonaticus

<220>
<221> misc_feature
<223> partial oriC locus

<400> 12
ggatcctggc tattaaaaag aagatctttt tatttagaga tctgttctat tgtgatctct 60

tattaggatc gccattggct gtggataagc cggatcccgg tattaagatc aataccttgg 120

taaggatcat tagctgtgaa tgatcggtga tcctggtccg tataagctgg gatcagaatg 180

aagggttata cacaactcaa aaactgaaca atggttattc tttggataac tactggttga 240

tccaaggttc ctgacagagt tatccacaca cgatcgcacg atctttacac 290

<210> 13
<211> 290
<212> DNA
<213> Salmonella typhimurium

<220>
<221> misc_feature
<223> partial oriC locus

<400> 13
ggatccgggc tattaaaaag aagatctttt tatttagaga tctgttctat tgtgatctct 60

tattaggatc gcgccaggct gtggataacc cggatcctgt aataaagatc aatgcgttgg 120

aaaggatcac tagctgtgaa tgatcggtga tcgtggtccg tataagctgg gatcaaaacg 180

ggtacttata cacaactcaa aaagtgaaca acggttattc tttggataac taccggttga 240

tccaagcttt ccaccagatt tatccacaat ggatcgcacg atctttacac 290

<210> 14
<211> 290
<212> DNA
<213> Citrobacter koseri

<220>
<221> misc_feature
<223> partial oriC locus

<400> 14
ggatcctggg tattaaaaag aagatctttt tatttagaga tctgttttat tgtgatctct 60

tattaggagc gcgattgtct gtggataacc aggatccgct gaataagatc aacaagttgt 120

tgaggatcat tagctgtgaa tgatcggtga tcctggcccg tataagctgg gatcagaatg 180

aagggttatg cacaactcaa aaactgaaca ccggttgttc tttggataac tactggttga 240

tccaagcttc ttaccagagt tatccacact cgatcgcacg atctttacac 290

<210> 15
<211> 290
<212> DNA
<213> Citrobacter murliniae

<220>
<221> misc_feature
<223> partial oriC locus

<400> 15
ggatcctggg tattaaaaag aagatctttt tatttagaga tctgttttat tgtgatctct 60

tattaggatc gcgatgttct gtggataagg gcgatcctct gaataagatc aacacgttgc 120

tgaggatcat taactgtgaa tgatcggtga tcctggtccg tataagctgg gatcaaaatg 180

aagggttata cacaactcaa aaactgaaca acggttgttc tttggataac taccggttaa 240

tccaagctta ctaccagagt tatccacact cgatcgcacg atctttacac 290

<210> 16
<211> 290
<212> DNA
<213> Citrobacter freundii

<220>
<221> misc_feature
<223> partial oriC locus

<400> 16
ggatcctggg tattaaaaag aagatctttt tatttagaga tctgttttat tgtgatctct 60

tattaggatc gcacctttct gtggataagg gcgatccttt aagtaagatc aacaggttgc    120

tgaggatcat tagctgtgaa tgatcggtga tcctggtccg tataagctgg gatcagaatg    180

aagggttata cacaactcaa aaactgaaca acggttgttc ttgggataac taccggttga    240

tccaagcttt taaccagagt tatccacata aaaaagtacg atctttacaa              290


<210>  17
<211>  290
<212>  DNA
<213>  Enterobacter aerogenes

<220>
<221>  misc_feature
<223>  partial oriC locus


<400>  17
ggatcctgag tattaaaaag aagatctttt atttagagat ctgttctatt gtgatctctt     60

attaggatcg actctctatg tggataagtc ggatccacga tttaagatca aacgcttaag    120

aaggatcaca atctgtgaat gatcggtgat cctgttccgt ataagctggg atcagaatga    180

agggttatgc acagctcaaa aagcatactc cggttattct ttggataact accggttgat    240

ccaagctttt gagcagggtt atccacagaa gaagctgcgg atctttacaa              290


<210>  18
<211>  289
<212>  DNA
<213>  Klebsiealla pneumoniae

<220>
<221>  misc_feature
<223>  partial oriC locus


<400>  18
ggatcctgag tgttaaaaag aagatctttt atatagagat ctgttctatt gtgatctctt     60

attaggatcg actctctttg tggataagtc ggatccgcga agtaagatca aaagcttaag    120

aaggatcact atctgtgaat gatcggtgat cctggtccgt ataagctggg atcagaatga    180

agggttatgc acagctcaaa aaccattcta cggttattct ttggataact accggttgat    240

ccaagctttt aagcatggtt atccacagaa gatcggacga tctttacaa               289


<210>  19
<211>  289
<212>  DNA
<213>  Klebsiella oxytoca

<220>
<221>  misc_feature
<223>  partial oriC locus

<400> 19
ggatcctgag tattaaaaag aagatctttt atttagagat ctgttttatt gtgatctctt 60

attaggatcg gcttgtcttg tggataagtc ggatccaaga tttaagatca atcgtttaag 120

aaggatcgct ttctgtgaat gatcggtgat cctggtccgt ataagctggg atcaaaatga 180

atggttatac acagcacaaa aatcagtcta cggttatact ttggataact accggttgat 240

ccaagctttt aagcatagtt atccacagaa gaaagtatga tctttacaa 289


<210> 20
<211> 289
<212> DNA
<213> Raoultella ornithinolytica

<220>
<221> misc_feature
<223> partial oriC locus


<400> 20
ggatcttgag tattaaaaag aagatctttt atttagagat ctgttttatt gtgatctctt 60

attaggatcg aacctttctg tggataagcc ggatccgcgt tttaagatca aatgcttaag 120

aaggatcact atctgtgaat gatcggtgat cctgttccgt ataagctggg atcaaaatga 180

agggttatac acaggtcaaa aagcagccac tggttatacg ttggataact accggttgat 240

cagagctttt aactgtagtt atccacagaa gatcggacga tctttacaa 289


<210> 21
<211> 289
<212> DNA
<213> Raoultella planticola

<220>
<221> misc_feature
<223> partial oriC locus


<400> 21
ggatcttgag tattaaaaag aagatctttt atttagagat ctgttttatt gtgatctctt 60

attaggatcg aacctttctg tggataagcc agatccgcgt tttaagatca aatgcttaag 120

aaggatcact atctgtgaat gatcggtgat cctgttccgt ataagctggg atcaaaatga 180

agggttatac acaggtcaaa aagcagccac tggttatacg ttggataact accggttgat 240

cagagctttt aagtgtagtt atccacagag gatcggacga tctttacaa 289


<210> 22
<211> 289
<212> DNA
<213> Raoultella terrigena

<220>
<221> misc_feature

<223>  partial oriC locus

<400>  22
ggatcttgag tatttaaaag aagatctttt atttagagat ctgttttatt gtgatctctt     60

attaggatcg gacctttctg tggataagcc ggatccgcgt tttaagatca atcggttaag    120

gaggatcact ttctgtgaat gatcggtgat cctgttccgt ataagctggg atcaaaatga    180

agggttatgc acagctcaaa aagcaggcaa cgcttatgcc ttggataact accggttgat    240

cagtggtttt aagcatagtt atccacagat gatcgggcga tctttacaa                289

<210>  23
<211>  290
<212>  DNA
<213>  Enterobacter intermedius

<220>
<221>  misc_feature
<223>  partial oriC locus

<400>  23
ggatcttggg tattaaaaag aagatctttt tatttagaga tctgttctat tatgatctct     60

tattaggatc gccaacttct gtggataagt gcgatccgcg atttaagatc aaccgtttaa    120

gaaggatcgt taactgtgaa tgatcggtga tcctggacag tataagctgg gatcaaaacg    180

aagggttata cacaggtcaa aaactaggca gaagttattc ttgggataac taccggttga    240

tcaatgcttt taaccagact tatccacaga ataaagtgcg atctttacaa               290

<210>  24
<211>  290
<212>  DNA
<213>  Kluyvera ascorbata

<220>
<221>  misc_feature
<223>  partial oriC locus

<400>  24
ggatcttggg tattaaaaag aagatctttt tatttagaga tctgttctat tgtgatctct     60

tattaggatc gccaaccttt gtggataagt ccgatccgga ttttaagatc aaccgtttaa    120

gaaggatcgt tagctgtgaa tgatcggtga tcctggtcag tataagctgg gatcaaaacg    180

aagggttatg cacaggtcaa aaaccagaca gaagttattc tttggataac taccggttga    240

tccaagcttt taaccagagt tatccacaca ataaattccg atctttacaa               290

<210>  25
<211>  289
<212>  DNA
<213>  Enterobacter gergoviae

<220>
<221>  misc_feature
<223>  partial oriC locus


<400>  25
cgatcctgag tattaaaaag aagatctttt aaagagagat ctgttttact gtgatctctt      60

attaggatcg ccttctcttg tggataagcc ggatcctgcg ttttggatca aatgcttatc     120

caggatcgta tgctgtgaat gatcggtgat cccgcaccgt ataagctggg atcagaatgc     180

ggagttatgc acaggacgaa aactagccaa accttatact ttggataact accggttgat     240

ccaagctttt aaccagagtt atccacagtt gatcgcacga tctttacac                 289


<210>  26
<211>  288
<212>  DNA
<213>  Enterobacter pyrinus

<220>
<221>  misc_feature
<223>  partial oriC locus


<400>  26
ggatcctgct taattaaaaa gaagatcttt taaagagatc tcttttactg tgatctctta      60

ttaggatcgg cttctcttgt ggataagtaa gatccggctt taaggatcat gtgtttaggc     120

aggatcgtta gctgtgaatg atcggtgatc ccggggcgta taagctgggg tcagaatgcg     180

gggttatgca caactcaaaa actaaccagc ccttatactt tggataacta ccggttgatc     240

caagctttta accagagtta tccacagaaa aagagacgat ctttacaa                  288


<210>  27
<211>  291
<212>  DNA
<213>  Enterobacter CDC

<220>
<221>  misc_feature
<223>  partial oriC locus


<400>  27
ggatcctggg ctattaaaaa gaagatcttt ttatttagag atctgttcta ttgtgatctc      60

ttattaggat cgcgtacttg tgtggataag tccgatccgg caaataagat catgcagtta     120

ggaaggatcg tttgctgtga atgatcggtg atcctggacc gtataagctg ggatcagatc     180

gcgactttat acacaggcta aaaacgatac aacagttgtt ctttggataa ctacgggtta     240

atacgagctt ttaaccagag ttatccacat ctgatcgcgc gatctttaat c              291


<210>  28

<211>  291
<212>  DNA
<213>  Enterobacter kobii

<220>
<221>  misc_feature
<223>  partial oriC locus


<400>  28
ggatcctggg ctattaaaaa gaagatcttt ttatttagag atctgttcta ttgtgatctc      60

ttattaggat cgcgtacttg tgtggataag tccgatccgg caaataagat catgcagtta     120

ggaaggatcg tttgctgtga atgatcggtg atcctggacc gtataagctg ggatcagatc     180

gcgactttat acacaggcta aaaacgatac aacagttgtt ctttggataa ctacgggtta     240

atacgagctt ttaaccagag ttatccacat ctgatcgcgc gatctttaat c              291


<210>  29
<211>  291
<212>  DNA
<213>  Enterobacer aspuriae

<220>
<221>  misc_feature
<223>  partial oriC locus


<400>  29
ggatcctggg ctattaaaaa gaagatcttt ttatttagag atctgttcta ttgtgatctc      60

ttattaggat cgcgaacttg tgtggataag tccgatctca taaataagat catgcagtta     120

gaaaggatcg tttgctgtga atgatcggtg atcctggaca gtataagctg ggatcagatc     180

gcgactttat acacaggtca aaaacgatac aacagttgtt ctttggataa ctacgggtta     240

atacgagctt ttaaccagag ttatccacat ctgttcgcgc gatctttaat c              291


<210>  30
<211>  291
<212>  DNA
<213>  Enterobacter sp. 25

<220>
<221>  misc_feature
<223>  partial oriC locus


<400>  30
ggatcctggg ctattaaaaa gaagatcttt ttatttagag atctgttcta ttgtgatctc      60

ttattaggat cgcggacacg tgtggataag tccgatcctg caaataagat catgcagtta     120

ggaaggatcg tttgctgtga atgatcggtg atccaggacc gtataagctg ggatcagatc     180

gcgactttat acacaggaca aaaacgacgc tagggttgtt ctttggataa ctacgggtta     240

atacgagctt ttaaccagag ttatccacat ctgttcgcgc gatctttaat c              291

<210> 31
<211> 291
<212> DNA
<213> Enterobacter cloacae

<220>
<221> misc_feature
<223> partial oriC locus

<400> 31
ggatcctggg ctattaaaaa gaagatcttt ttatttagag atctgttcta ttgtgatctc 60

ttattaggat cgcggacctt tgtggataag tccgatctca taaataagat catggggtta 120

gaaaggatcg tttgctgtga atgatcggtg atcctggccc gtataagctg ggatcagatc 180

gcgactttat acacagggca aaaacgactt caacgttgtt ctttggataa ctactgctta 240

atacgagctt ttaaccagag ttatccacat ctgttcgcgc gatctttaat c 291

<210> 32
<211> 291
<212> DNA
<213> Enterobacter dissolvens

<220>
<221> misc_feature
<223> partial oriC locus

<400> 32
ggatcctggg ctattaaaaa gaagatcttt ttatttagag atctgttcta ttgtgatctc 60

ttattaggat cgaggacctt tgtggataag tccgatctca taaagaagat catggagtta 120

gaaaggatcg tttgctgtga atgatcggtg atcctggccc gtataagctg ggatcagatc 180

gcgactttat gcacaggcca aaaacgactt caccgttgtc ctttggataa ctactgctta 240

atacgagctt ttaaccagag ttatccacat ctgttcgcgc gatctttaat c 291

<210> 33
<211> 291
<212> DNA
<213> Enterobacter sp. 114

<220>
<221> misc_feature
<223> partial oriC locus

<400> 33
ggatcctggg ctattaaaaa gaagatcttt ttatttagag atctgttcta ttgtgatctc 60

ttattaggat cgagtacctc tgtggataag cccgatctca cgatgaagat catgcagtta 120

gaaaggatcg tttgctgtga atgaacggtg atcctggcca gtataagctg ggatcagatc 180

gcgactttat acacaggccc aaaacagcct cagagttata acttggataa ctaccgctta     240

atacgagctt ttaaccagag ttatccacat ctgttcgcgc gatctttaat c              291

<210>  34
<211>  291
<212>  DNA
<213>  Enterobacter sp. 18

<220>
<221>  misc_feature
<223>  partial oriC locus

<400>  34
ggatcttggg ctattaaaaa gaagatcttt ttatttatag atctgttcta ttgtgatctc      60

ttattaggat cgcagacctt tgtggataag tccgatccca taaataagat catgcagtta     120

ggaaggatcg tttgctgtga atgatcggtg atcctggacc gtataagctg ggatcagatc     180

gctactttat acacaggtca aaaactaaac caacgttgtt ctttggataa ctacggctta     240

atacgagctt ttaaccagag ttatccacat ctgatcgcac gatctttaat c              291

<210>  35
<211>  291
<212>  DNA
<213>  Enterobacter hormaechei

<220>
<221>  misc_feature
<223>  partial oriC locus

<400>  35
ggatcctggg ctattaaaaa gaagatcttt ttatttagag atctgttcta ttgtgatctc      60

ttattaggat cgcagacctt tgtggataag tccgatccca taaataagat catgcagtta     120

ggaaggatcg tttgctgtga atgatcggtg atcctggacc gtataagctg ggatcagatc     180

gcccctttat ccacaggtca aaaactaagt caacgttatt ctttggataa ctacggctta     240

atacgagctt ttaaccagag ttatccacat ctgatcgcac gatctttaat c              291

<210>  36
<211>  291
<212>  DNA
<213>  Enterobacter sp. 36

<220>
<221>  misc_feature
<223>  partial oriC locus

<400>  36
ggatcctggg ctattaaaaa gaagatcttt ttatttagag atctgttcta ttgtgatctc      60

ttattaggat cgcagacctt tgtggataag tccgatccca taaataagat catgcagtta     120

ggaaggatcg tttgctgtga atgatcggtg atcctggacc gtataagctg ggatcagatc 180

gccactttat acacaggtca aaaactaaac taacgttgtt ctttggataa ctacgggtta 240

atacgagctt ttaaccagag ttatccacat ccgatcgcac gatctttaat c 291

<210> 37
<211> 291
<212> DNA
<213> Enterobacter cancerogenes

<220>
<221> misc_feature
<223> partial oriC locus

<400> 37
ggatcctggg ctattaaaaa gaagatcttt ttatttagag atctgttcta ttgtgatctc 60

ttattaggat cgcggacctt tgtggataag cccgatctca cgaagaagat catgcgttta 120

agaaggatcg ttagctgtga atgatcggtg atcctggacc gtataagctg ggatcagatc 180

gcgtatttat ccacagctca aaaacgaagc agcggttgtt atttggataa ctaccggtta 240

atacgagctt ttaaccagag ttatccacat ctgtttgcgc gatctttaat c 291

<210> 38
<211> 290
<212> DNA
<213> Enterobacter amnigenus

<220>
<221> misc_feature
<223> partial oriC locus

<400> 38
ggatcctggc tattaaaaag aagatcttct tatttagaga tctgttttac tatgatctct 60

tattaggatc gcacgtttct gtggataagc gggatctcat caggaagatc ataacgttag 120

aaaggatcgt ttgctgtgaa tgatcggtga tcctggacag tataagctgg ggttagatcg 180

tgactttatc cacagctcaa aaaggaagca agcgttatac attggataac taccgcttaa 240

tccaagcttt taaccagact tatacacatc tgatcgcgtg atctttaatc 290

<210> 39
<211> 290
<212> DNA
<213> Enterobacter nimipressuralis

<220>
<221> misc_feature
<223> partial oriC locus

<400> 39

ggatcttggc tattaaaaag aagatcttct tatttagaga tctgttttac tatgatctct 60

tattaggatc gccttctcgt gtggataagt cggatccggt cataaagatc ataaggatag 120

aaaggatcgt ttgctgtgaa tgatcggtga tcctggaccg tataagctgg ggttagatcc 180

gctacttata cacaggtcaa aaaccagcca acggttatac cttggataac taccggttga 240

tccaaggttt taaccagact tatccacatc tgatcgcacg atctttaagc 290

<210> 40
<211> 291
<212> DNA
<213> Enterobacter cowanii

<220>
<221> misc_feature
<223> partial oriC locus

<400> 40
ggatcccggc tattaaaaag aagatcttta tatagagaga tctgttctat tgtgatctct 60

tattaggatc gcgatgtctt gtggataagc cggatcatcg ctttaagatc agttcattag 120

gaaggatcat taactgtgaa tgatcggtga tcctgcaacg tataagctgg gatcaaaagg 180

gctagttata cacagggcgg atcggtgagc agagttattc attggataac taccggttaa 240

taagcgcttt taagcctagt tatcccactg ctgatcgcac gatctttaca c 291

<210> 41
<211> 289
<212> DNA
<213> Enterobacter vulneris

<220>
<221> misc_feature
<223> partial oriC locus

<400> 41
ggatcctgac tattaaaaga agatcttttt tatttaagat ctgttttatt atgatctctt 60

attaggagcg ccatctcctg tggataaggt ggatcctctt attaagatca acgtgttatt 120

gaggatcgtt tgctgtgaaa gatcggtgat cccacaccgt ataagctggg atcaaaaagg 180

cgggttatac acaggcgaaa ctttaaacaa accttataca gtggataact accggttgat 240

caacgctttt aaccagagtt acccacagct gatcgcacga tctttacac 289

<210> 42
<211> 289
<212> DNA
<213> Enterobacter sakazakii

<220>
<221> misc_feature
<223> partial oriC locus

<400> 42
ggatcgttgg tattaaaaag aagatctttt atctaaagga tctgttttat tatgatctct 60

tattaggatc gacatccctt gtggataacc cggatcgccc atttgggatc aaaaggttat 120

ccgggatcgc ttgctgtgaa tgatcggtga tcccagaccg tataagctgg gatcagaaat 180

gagggttata cacaactcaa aaagcgaaca acacttatac cttggataac tacgggttga 240

tccaagcttt taagctcttt tatccacagc tgaatggcga tcatttcgc 289

<210> 43
<211> 289
<212> DNA
<213> Enterobacter hermanii

<220>
<221> misc_feature
<223> partial oriC locus

<400> 43
ggatcgttgg taattaaaag aagatctttt tatttagaga tctgttttac tgtgatctct 60

tattaggatc gccgttatct gtggataacc gcagatcgtt aattaagatc aatacattaa 120

gcgggatcat tagctgttaa cgatcggtga tcccacacgg tattagctgg gtgcagaaag 180

ggtatttata cacagggcca aaaagtcaaa aaggttatac gttggataac taccggttga 240

tccaagctta tgagcagact tatccacatt caaccggcga tcttttcga 289

<210> 44
<211> 290
<212> DNA
<213> Buttiauxella agrestis

<220>
<221> misc_feature
<223> partial oriC locus

<400> 44
ggatcgtcgg taattaaaag aagatctttt ttatttaaag atctctttat tatgatctct 60

tattaggatc ggcatgttct gtggataacc gtttttatgg taaagagatc aagtgattaa 120

ggaggatcat tagctgtgaa tgatcggtga tcccagaacg tataagctgg gatcaaaagt 180

ggaagttatg cacaactcaa aaagtgaaca ggagttgtta ttgggataac tactgcttaa 240

cccaaggttt taagcatact tatacacaga taaaagtcag atctttacaa 290

<210> 45
<211> 291
<212> DNA
<213> Cedecea neteri

<220>
<221>  misc_feature
<223>  partial oriC locus


<400>  45
ggatcttcgg taattaaaag aagatctttt ttatttaaag atctctttat tatgatctct      60

tattaggatc gaccacccct gtggataagc catttatcca ttttaggatc aacaacctgg     120

aaaggatcgt ttgctgtgta ctaccagtga tcccagaccg tataagctgg gatcagaatt     180

gaggtttatg cacaactcaa aaagtgaaca gcagttgtta ttgggataac taccgcttaa     240

tccaagcttt taaccagagt tatccacatc tgatcgtgca atcctttaca c              291


<210>  46
<211>  288
<212>  DNA
<213>  Pectobacterium carotovorum

<220>
<221>  misc_feature
<223>  partial oriC locus


<400>  46
ggatcctcgt taattaaaga aagatctttt tatttaaaga tctctttatt agatctctta      60

ttaggatcgt catgttgtgt ggataagtgg ttattcatat taaagatcaa gagaatggcg     120

ttgatcctta cctgtgaatg atcggtgatc ctggacagta taagctggga tcttaatgcg     180

ggtttatgca caggacaaaa aacatacttg ggttgttatt gggataacta cggcttttac     240

acctgaatta agcatagtta tccacattca actgctgaat atttcatc                  288


<210>  47
<211>  288
<212>  DNA
<213>  Yersinia pestis

<220>
<221>  misc_feature
<223>  partial oriC locus


<400>  47
ggatcgcctt taattaaaag aagatctttt tatttaaaga tctctttatt agatctctta      60

ttaggatcat gatcctctgt ggataagtga ttattcacat ggaagatcat ataattaagg     120

aggatcgttt gttgtgagtg accggtgatc ctattgcgta taagctggga tctaaatggc     180

atgttatgca cagtcactcg gcagaatcaa ggttgttatg tggatatcta ctggttttac     240

cctgctttta agcatagtta tacacattcg ttcgcgcgat ctttgagc                  288


<210>  48
<211>  290

<212>  DNA
<213>  Pseudomonas aeroginosa

<220>
<221>  misc_feature
<223>  partial oriC locus


<400>  48
gacgtaatag atagatacaa ggaagtcatt tttcttttaa aggatagaaa cggttaatgc      60

tcttgggacg gcgcttttct gtgcataact cgatgaagcc cagcaattgc gtgtttctcc     120

ggcaggcaaa aggttgtcga gaaccggtgt cgaggctgtt tccttcctga gcgaagcctg     180

gggatgaacg agatggttat ccacagcggt tttttccaca cggctgtgcg cagggatgta     240

cccccttcaa agcaagggtt atccacaaag tccaggacga ccgtccgtcg               290


<210>  49
<211>  290
<212>  DNA
<213>  Pseudomonas putita

<220>
<221>  misc_feature
<223>  partial oriC locus


<400>  49
cggatcgggg acaacatgaa aaaaaagaag agacatataa aaagcttttt tgaagaactt      60

ataactctta agtggataac cttctgtgga taacctgcgc tggcccatga attacggggt     120

gtacagagtt ttacaacttt gttctgatcc cgtgctgcgc ttgttccaat cgtgagcgaa     180

agctgtggat gaaaacacct gttatccaca gcggagttat caacaggcta aggggtgggg     240

ttgtgcatag ccctcatggt cgtttatcca cagggcttat tcacagaggc               290

## Description

The present invention is related to nucleic acid molecules suitable for the detection of coliforms and in particular *Escherichia. coli,* combinations of such nucleic acid molecules, methods for detecting and/or identifying and/or quantifying these bacteria and kits suitable for such purpose.

*Enterobacteriaceae* is a group of Gram negative bacteria and comprises quite a number of pathogenic organisms. These pathogenic organisms are relevant in the field of medicine and hygiene as well as the food indentry including water quality control.

The members of the *Enterobacteriaceae* family are closely related but in itself very heterogeneous in terms of their biochemical characteristics. Due to the potential risk arising from the presence of *Enterobacteriaceae* or some members thereof and in view of the afore-mentioned heterogeneity of the genus *Enterobacteriaceae* in terms of biochemical characteristics which are usually used to characterize this group of microorganisms, a group of indicator bacteria referred to as coliforms was defined. The presence of any organism belonging to this group of bacteria is regarded as an indication for the presence of human and animal faeces which inherently bears the risk that also potentially pathogenic organisms are present. Due to this correlation, a specific detection of any actually pathogenic organism which is usually time consuming and expensive, is usually not required. The detection of coliforms is sufficient to trigger distinct measures suitable to protect man, animals and the environment, whereby the individual measures vary, depending on the kind of sample analyzed and on the location from which the respective sample had been taken. A particularly important field where the concept of indicator organisms is of tremendous importance, is the hygienic control of water and in particular drinking water and tap water.

In the art several approaches have been developed and are currently used to detect coliforms. One of said approaches is based on the biochemical traits of an individual single-cell based colony. Although the most traditional one, however, this approach is also the one most prone to false results due to the above mentioned metabolic heterogeneity of this closely related group of bacteria where the biochemical traits typically do not go along with species boundaries. Factors contributing to the observed heterogeneity of biochemical traits within the family of *Enterobacteriaceae* are, among others, gene transfer within this group, mutations in the genes of metabolic enzymes as well as occurence of biotypes which are rear and have not yet properly been investigated. In view of this, the biochemical characterisation of bacteria to decide whether they belong to the group of coliforms is only a first measure which rarely reaches a sensitivity and specificity level, respectively, of more than 95%. Apart from that, the cultivation and biochemical characterisation typically requires about 48 hours.

A further approach for detecting coliforms and *E. coli* in particular, is based on specific DNA sequences. However, the lack of species- and group-specific DNA sequences did not allow to fully exploit any technique based thereon (Rompre et al., J. Microbiol. Methods. 2002, Mar; 49(1) : 31-54). Among others, it is proposed to detect *E. coli* by using specific DNA probes directed to 16S rRNA sequences, the β-D-glucuronidase gene and other genes like *lacZ, lamb* and *phoE.*

The reason why in particular ribosomal gene sequences are not suitable for the identification of individual species within the family of *Enterobacteriaceae,* resides in the too close relationship between the members of the family and that the ribosomal genes do not exhibit significant differences. Additionally, repetitive gene sequences are exchanged between the members of the *Enterobacteriaceae* family which does not allow to use this kind of nucleic acid based information in the identification or detection of *Enterobacteriaceae.*

A still further approach makes use of protein encoding genes. Also this technique is not really suitable to characterize groups or individual species within the *Enterobacteriaceae* family as the distribution of the various genes is not bound to taxonomic boundaries. Additionally, quite frequently different alleles exist within a single species which would require to test for numerous genotypes and biotypes.

Due to the disadvantages associated with any of these newer methods, for the time being no assay based thereon has been acknowledged and authorized by the health authorities for medical diagnosis and water quality monitoring.

Therefore, one problem underlying the present invention is to provide a target which allows for both a sequence- and group-specific detection within the *Enterobacteriaceae* family. A further problem underlying the present invention is to provide a target or target nucleic acid which allows a species-specific and/or group-specific detection within the *Enterobacteriaceae* family, whereby one group within the *Enterobacteriaceae* family which is to be detected in a group-specific manner is the group of coliforms. Finally, a problem underlying the present invention is to provide a target which allows a species- and/or group-specific detection within the *Enterobacteriaceae* family covering the various genotypes and biotypes of the respective species and groups, preferably the genotypes and biotypes of *E. coli* and the group of coliforms.

A still further problem underlying the present invention is to provide means to detect bacteria of the group of coliforms and bacteria of the *E. coli* genogroup and in particular *E. coli,* and more specifically to differentiate *E. coli* and bacteria of the *E. coli* genogroup within the group of coliforms.

These problems are solved by the subject matter of the independent claims. Preferred embodiments may be taken from the dependent claims.

More specifically, the problem underlying the present invention is solved in a first aspect by a combination of at least a first nucleic acid molecule and at least a second nucleic acid molecule for the detection of and/or discrimination between bacteria of the group of coliforms and bacteria of the *Escherichia coli* genogroup, whereby the first nucleic acid and the second nucleic acid differ in their nucleic acid sequence and comprise fragments of the origin of replication *(oriC)* locus sequence of said bacteria.

In an embodiment the first and the second nucleic acid molecule each and independently have a length of at least 14 or 15 nucleotides.

In an embodiment the first and the second nucleic acid molecule each and independently have a maximum length of 600 nucleotides, preferably 300, more preferably 50 and most preferably 30 nucleotides.

According to the present invention the problem is solved in a second aspect by a combination of at least a first nucleic acid molecule and a second nucleic acid molecule, preferably according to the first aspect of the present invention, whereby the first nucleic acid molecule is selected from the group comprising
a nucleic acid molecule comprising the following nucleic acid sequence:
5'-CAAAAGGATCCKGATAAAACATGGT-3' (SEQ. ID. No. 1),
a nucleic acid molecule comprising the following nucleic acid sequence:
5'-CGGGCCGTGGATTCTACTCA-3' (SEQ. ID. No. 2), and
a nucleic acid molecule comprising the following nucleic acid sequence:
5'-CATGAGATTCCGGARGATCCG-3' (SEQ. ID. No. 3); and
whereby the second nucleic acid molecule is selected from the group comprising
a nucleic acid molecule comprising the following nucleic acid sequence:
5'-CACTGCCCTGTGGATAACAA-3' (SEQ. ID. No.4), and
a nucleic acid molecule comprising the following nucleic acid sequence:
5'-TATACAGATCGTGCGATCTAC-3' (SEQ. ID. No. 5).

In an embodiment the first nucleic acid molecule is specific for the group of coliforms.

In a preferred embodiment the group of coliforms comprises *E. coli, E. fergusonii, Enterobacter* spp., *Klebsiella* spp., *Citrobacter* spp., *Raoutella* spp., *Salmonella* spp., and *Shigella* spp.

In an embodiment of the first and the second aspect the second nucleic acid molecule is specific for the *E. coli* genogroup.

In a preferred embodiment of the first and the second aspect the *E. coli* genogroup comprises *E. coli, E. fergusonii* and *Shigella* spp.

In an embodiment of the first and the second aspect the first nucleic acid molecule is a combination of a nucleic acid molecule comprising a nucleic acid sequence according to SEQ.ID.No. 1 and a nucleic acid molecule comprising a nucleic acid sequence according to SEQ.ID.No. 3, or a combination of a nucleic acid molecule comprising a nucleic acid sequence according to SEQ.ID.No. 2 and a nucleic acid molecule comprising a nucleic acid sequence according to SEQ.ID.No. 3.

In an embodiment of the first and second aspect the second nucleic acid molecule is a combination of a nucleic acid molecule comprising a nucleic acid sequence according to SEQ.ID.No. 4 and a nucleic acid molecule comprising a nucleic acid sequence according to SEQ.ID.No. 5

In an embodiment of the first and second aspect each and any of the nucleic acid molecules is individually and independently labelled.

In an embodiment of the first and second aspect the label is selected from the group comprising radioactive labels, fluorescent labels, oligonucleotides and haptens.

According to the present invention the problem is solved in a third aspect by a nucleic acid molecule, whereby the nucleic acid molecule is selected from the group comprising
a nucleic acid molecule comprising the following nucleic acid sequence:
5'-CAAAAGGATCCKGATAAAACATGGT-3' (SEQ. ID. No. 1),
a nucleic acid molecule comprising the following nucleic acid sequence:
5'-CGGGCCGTGGATTCTACTCA-3' (SEQ. ID. No. 2), and
a nucleic acid molecule comprising the following nucleic acid sequence:
5'-CATGAGATTCCGGARGATCCG-3' (SEQ. ID. No. 3).

In an embodiment of the third aspect the nucleic acid molecule is for the identification and/or detection and/or quantification of bacteria, preferably coliforms.

In an embodiment of the third aspect the nucleic acid molecule is used as a primer and/or as a probe.

According to the present invention the problem is solved in a fourth aspect by a nucleic acid molecule, whereby the nucleic acid molecule is selected from the group comprising
a nucleic acid molecule comprising the following nucleic acid sequence:
5'-CACTGCCCTGTGGATAACAA-3' (SEQ. ID. No.4), and
a nucleic acid molecule comprising the following nucleic acid sequence:
5'-TATACAGATCGTGCGATCTAC-3' (SEQ. ID. No. 5).

In an embodiment of the fourth aspect the nucleic acid molecule is for the identification and/or detection and/or quantification of bacteria, preferably bacteria of the E. *coli* genogroup.

In an embodiment of the fourth aspect the nucleic acid molecule is used as a primer and/or as a probe.

According to the present invention the problem is solved in a fifth aspect by a method using probes or amplification primers or both for determining the presence or amount of nucleic acid, whereby the nucleic acid is from bacteria of the group of coliforms or from bacteria of the E. *coli* genogroup, whereby the probes or amplification primers are derived from bacteria of the *oriC* locus of the group of coliforms or from bacteria of the E. *coli* genogroup.

In an embodiment of the fifth aspect the probes or amplification primers comprise a nucleic acid sequence according to SEQ.ID.No. 1, SEQ.ID.No. 2 or SEQ.ID.No. 3 and are preferably used for determining the presence or amount of bacteria of the group of coliforms or a nucleic acid thereof.

In an embodiment of the fifth aspect the probes or amplification primers comprise a nucleic acid sequence according to SEQ.ID.No. 4 or SEQ.ID.No. 5 and are preferably used for determining the presence or amount of bacteria of the E. *coli* genogroup or a nucleic acid thereof.

According to the present invention the problem is solved in a sixth aspect by a method for the detection and/or identification and/or quantification of bacteria of the group of coliforms in a sample, comprising the step of
- detecting a nucleic acid fragment of the *oriC* locus of the group of coliforms.

In an embodiment of the sixth aspect a nucleic acid molecule according to the third aspect of the invention is used in the step of detecting a nucleic acid fragment.

In an embodiment of the sixth aspect the nucleic acid fragment of the *oriC* locus is amplified in a step using a nucleic acid molecule according to the third aspect of the invention prior to the detection step.

According to the present invention the problem is solved in a seventh aspect by a method for the detection and/or identification and/or quantification of bacteria of the *E. coli* genogroup, comprising the step of
- detecting a nucleic acid fragment of the *oriC* locus of bacteria of the *E. coli* genogroup.

In an embodiment of the seventh aspect a nucleic acid molecule according to the fourth aspect of the invention is used in the step of detecting a nucleic acid molecule.

In an embodiment the nucleic acid fragment of the *oriC* locus is amplified in a step using a nucleic acid molecule according to the fourth aspect of the invention prior to the detection step.

According to the present invention the problem is solved in a eighth aspect by a method for differential detection and/or differential identification and/or differential quantification of bacteria of the group of coliforms and of bacteria of the *E*. *coli* genogroup in a sample, comprising the steps of
a) providing a nucleic acid preparation of the sample, and
b) contacting a combination of at least a first nucleic acid molecule and a second nucleic acid molecule according to the first and/or the second aspect of the invention with the nucleic acid preparation.

In an embodiment of the eighth aspect the method further comprises as step
c) detecting in the nucleic acid preparation a nucleic acid molecule which or a part of which is either essentially identical to or essentially complementary to the first and/or the second nucleic acid molecule.

In an embodiment of the eighth aspect the bacteria of the group of coliforms is detectable by the first nucleic acid molecule and the group of bacteria of the *E. coli* genotype is detectable by the second nucleic acid molecule.

In an embodiment of the eighth aspect the sample is analysed in a first step for bacteria of the group of coliforms by using the first nucleic acid molecule and in a second step for bacteria of the *E. coli* genogroup using the second nucleic acid molecule.

In a preferred embodiment of the eighth aspect the bacteria identified as belonging to the group of coliforms in the first step are analysed in the second step whether they belong to the group of bacteria of the *E. coli* genotype.

In an embodiment of the eighth aspect the first and/or the second nucleic acid molecule is independently and individually used as primers and/or as probes.

In an embodiment of the eighth aspect the first nucleic acid molecule is a combination of a nucleic acid molecule comprising a nucleic acid sequence of SEQ.ID.No. 1 and a nucleic acid molecule comprising a nucleic acid sequence of SEQ.ID.No. 3, or a combination of a nucleic acid molecule comprising a nucleic acid sequence of SEQ.ID.No. 2 and SEQ.ID.No. 3.

In an embodiment of the eighth aspect the second nucleic acid moecule is a combination of a nucleic acid molecule comprising a nucleic acid sequence of SEQ.ID.No. 4 and a nucleic acid molecule comprising a nucleic acid sequence of SEQ.ID.No. 5.

In an embodiment of the eighth aspect the nucleic acid preparation is amplified in a step using the first and/or the second nucleic acid molecule.

In an embodiment of the sixth to eighth aspect the step of amplification comprises the practicing of a method selected from the group comprising polymerase chain reaction, ligase chain reaction, nucleic acid sequence-based amplification, self-sustained sequence replication, strand displacement amplification, branched DNA signal amplification, nested PCR, and multiplex PCR.

In an embodiment of the sixth to eighth aspect the sample is obtained from patients, preferably human patients, animals, environment or food.

In a preferred embodiment of the sixth to eighth aspect the sample is a water sample.

According to the present invention the problem is solved in a ninth aspect by a kit for the detection and/or identification and/or quantification of bacteria of the group of coliforms comprising at least one nucleic acid according to the third aspect of the invention.

According to the present invention the problem is solved in a tenth aspect by a kit for the detection and/or identification and/or quantification of bacteria of the group of *E. coli* genotype comprising at least one nucleic acid according to the fourth aspect of the invention.

According to the present invention the problem is solved in a eleventh aspect by a kit for the differential detection and/or differential identification and/or differential quantification of bacteria of the group of coliforms and of bacteria of the *E.* coli genogroup comprising at least one nucleic acid according to the third aspect of the invention and at least one nucleic acid according to the fourth aspect of the invention.

The present invention is based on the surprising finding of the inventor that the origin of replication *(oriC)* locus of *Enterobacteriaceae* is a suitable target or target nucleic acid as used in a synonymous manner herein, which allows to provide specific means such as, e.g., probes and primers for detecting bacteria of the *E. coli* genogroup on the one hand and bacteria of the group of coliforms on the other hand as respective groups. More particularly, the means according to the present invention are specific in terms of each detecting the bacteria of the *E. coli* genogroup or the bacteria of the group of coliforms without the need to have to use individual means for the detection or identification of each member of said groups. Because of this, the concept of indicator bacteria can be further applied using two different means, one means being specific for the *E. coli* genogroup and the other means being specific for bacteria of the group of the coliforms.

The differences between the *oriC* loci of the *Enterobacteriaceae* have been found to be sufficiently well expressed so as to allow this kind of discrimination between said two groups of bacteria within the *Enterobacteriaceae* family. Insofar, a reliable differential diagnosis within the family of *Enterobacteriaceae* and other microorganisms using a molecular marker is now possible which allows for a rapid detection, identification and quantification, respectively, of said bacteria of said two groups of bacteria in particular.

Additionally, the present inventor has surprisingly found that the relationship between the various members of the *Enterobacteriaceae* family is reflected by the degree of homology in the *oriC* locus. Insofar, the *oriC* locus provides for the required degree of conservacy needed for the identification of individual species and in particular of the two groups of bacteria, namely bacteria of the *E. coli* genotype and bacteria of the group of coliforms. Despite the homogeneity in the *oriC* locus within the *Enterobacteriaceae,* the various biotypes and genotypes do not have to be characterized in terms of *oriC* heterogeneity locus so that the use of respective *oriC* locus based or *oriC* locus targeting nucleic acid molecules such as probes and primers is suitable for identifying, detecting and quantifying these organisms and group of organisms, respectively. Without whishing to be bound by any theory, the present inventor assumes for the time being that the *oriC* locus is also a particularly suitable target as the *oriC* locus can not be transferred between bacteria and is a single copy gene which is furthermore obligatory for the gene locus. Additionally, no different alleles could be observed in the *oriC* locus within a single species.

As preferably used herein, the term *E. coli* genogroup comprises *E. coli, Shigella* spp. and *E. fergusonii.*

As also preferably used herein, the term bacteria of the group of coliforms comprises *E. coli, E. fergusonii, Enterobacter* spp., *Klebsiella* spp., *Citrobacter* spp., *Raoultella* spp., *Salmonella* spp. and *Shigella* spp. Further members, species and strains of said two groups can be taken from the table X mentioned in the example part of the present specification.

Within the *oriC* locus of the *Enterobacteriaceae* family which typically comprises about 600 bp with a core region comprising about 235, 261 and 290 bp, respectively, the terminal stretches thereof are particularly suitable for defining species- and group-specific nucleic acid molecules. Such species- and group-specific nucleic acid molecules are also referred to herein as the nucleic acid molecules according to the invention.

As preferably used herein, the oriC locus is defined by binding sites for proteins involved in cell division such as DnaA, FIS and IHF and can be identified based thereon by the ones skilled in the art for each and any species or group of bacteria.

Particularly preferred nucleic acid molecules according to the invention are those which allow for the detection of bacteria of the *E. coli* genogroup, whereby the nucleic acid molecule preferably is a nucleic acid molecule comprising a nucleic acid sequence of SEQ.ID.No.4 or SEQ.ID.No. 5 or a combination thereof.

Particularly preferred nucleic acid molecules according to the invention are those which allow for the detection of bacteria of the group of coliforms, whereby the nucleic acid molecule preferably is a nucleic acid molecule comprising a nucleic acid sequence of SEQ.ID.No1, 2 or 3 or any combination thereof.

Apart from the nucleic acid molecules comprising a nucleic acid sequence according to any of SEQ.ID.Nos. 1-5, the nucleic said molecules according to the invention also comprise nucleic acid molecules which interact with or detect the same locus or loci as detectable by the nucleic acid molecules having or comprising the sequences according to SEQ.ID.No. 1-5. In a preferred embodiment, such nucleic acid molecules differ from the particular sequences disclosed herein by one or more nucleotides. Although this kind of nucleic acid molecules is not perfectly base-pairing according to Watson-Crick base-pairing rules, a specific interaction with the target nucleic acid which is preferably the *oriC* locus, more preferably the part of the *oriC* locus which is specific for the bacteria of the *E. coli* genogroup and for the bacteria of the group of coliforms, respectively, and even more preferably the corresponding part of the *oriC* locus which is specifically addressed by the nucleic acid molecules according to the present invention having a nucleic acid sequence according to any of SEQ.ID.Nos.1 to 5, can still be observed or generated if the hybridisation conditions are chosen such that the specificity and sensitivity and in particular the specificity for the group of bacteria of the *E. coli* genogroup and for the group of bacteria of the group of coliforms, respectively, is still assured. The nucleic acid molecules according to the present invention also comprise nucleic acid molecules which are complementary to the nucleic acid molecules described herein, preferably complementary to the nucleic acid molecules having a nucleic acid sequence according to any of SEQ.ID.Nos. 1 to 5.

As used herein the term "a nucleic acid molecule comprising a nucleic acid sequence" preferably means either that the nucleic acid molecule has the nucleic acid, i.e. consists thereof, or that the nucleic acid molecule comprises the nucleic acid sequence, where by the sequence is not limited to the particular one but comprises a further sequence. In the latter case the further sequence can be any sequence under the proviso that such further sequence would not interfere with the use or application of the nucleic acid molecule having the nucleic acid sequence only. The further sequence may, therefore, be a sequence which is copied from the target nucleic acid molecule, i.e. the oriC locus or adjacent nucleic acid stretche(s), whereby such copying occurs and such further sequence arises from the use of a nucleic acid molecule according to the present invention as a primer, particularly as a primer in an amplification reaction. In a respective embodiment, the length of the thus extended primer and thus the length of the nucleic acid molecule used or useful for the various applications described herein for the nucleic acids and nucleic acid molecule combinations, respectively, may range from 14 to 600 nucleotides. From such ranges it is evident that the detected nucleic acid molecule is not necessarily limited to the oriC locus, preferably upon extension of the nucleic acid molecule(s) according to the present invention.

It is also within the present invention that the term "fragment of the oriC locus" also comprise the full length ori C locus or parts thereof.

The terms nucleic acid and nucleic acid molecule are used herein in an interchangeable manner if not indicated to the contrary.

The nucleic acid molecules according to the present invention also comprise nucleic acid molecules having a homology of at least 70 %, preferably at least 80 % and more preferably at least 90 % compared to the nucleic acid molecules according to the present invention having a nucleic acid sequence according to any of SEQ.ID.Nos. 1 to 5 or nucleic acid molecules having a nucleic acid sequence which is complementary thereto.

It will be acknowledged that the nucleic acid molecules according to the present invention also comprise such molecules having the aforementioned homology.

As may also be taken from the example part, the nucleic acid molecule having the nucleic acid sequence according to SEQ.ID.No. 4 differs from the most closely related species by six mismatches, whereas the nucleic acid molecule according to SEQ.ID.No. 5 differs from the most closely related species by four mismatches. Under appropriate hybridisation conditions, this degree of difference between the target nucleic acid of the *oriC* locus and the nucleic acid molecule according to the invention having the nucleic acid sequences of SEQ.ID.No. 4 and 5, expressed as mismatches, can actually be either increased or decreased. In any case, the effect of such mismatches can be compensated by increasing or decreasing the stringency of the hybridisation conditions realized upon using the nucleic acid molecules according to the present invention. The change in stringency of the hybridisation conditions which has to be made so as to allow the specific detection, identification and/or quantification of bacteria of the E. *coli* genogroup and of bacteria of the group of coliforms using such degenerated nucleic acid molecules, i. e. nucleic acid molecules which have or comprise a nucleic acid sequence differing from the sequences according to SEQ.ID.Nos 1 to 5 but still allowing such specific detection, identification and/or quantification of said two groups of bacteria, is obvious for the one skilled in the art and can be determined by using standard calculations and routine experiments. Such degenerated nucleic acid molecules according to the present invention are also referred to herein as essentially identical or essentially complementary nucleic acid molecules. Appropriate hybridisation conditions particularly for using the nucleic acid molecules comprising or having a nucleic acid sequence according to any of SEQ.ID.Nos 1 to 5 are those as described in the example part of the present specification. In connection therewith it is to be noted that the hybridisation conditions can be varied as known by the ones skilled in the art. If the temperature is change under the hybridisation regimen described herein, preferably the change or deviation from the values set forth herein, is typically 2°C or less, preferably 1°C and more preferably 0.5°C.

It will be acknowledged that using the nucleic acid molecules according to the present invention and in particularly those having SEQ.ID.No. 1 to 3 and nucleic acid molecules complementary thereto as well as degenerated nucleic acid molecules thereof allows for the detection of the bacteria of the group of coliforms and for the specific detection of the reference species strains of the coliforms, and in particular those having SEQ.ID.No. 4 and 5 and nucleic acid molecules complementary thereto as well as degenerated nucleic acid molecules thereof allow for the specific detection of bacteria of the *E.* coli genogroup and for the specific detection of the reference species strains of the bacteria of the *E. coli* genogroup.

It is within the present invention that this kind of nucleic acid molecule may comprise further constituents. Said constituents may have different functions. One function can be for handling of the molecules, another function can be for the detection of the molecules. It will be acknowledged that some of the constituents used for one purpose may also serve the other or another purpose.

Particularly for detection purposes, the nucleic acid molecules according to the present invention may comprise one or several fluorescence labels, chemoluminescence labels, radioactive labels, some enzymatically active groups, haptens or nucleic acid detectable by hybridisation, whereby such nucleic acid is different from a nucleic acid which is essentially identical or essentially complementary to the nucleic acid molecules according to the present invention. It is within the present invention that a nucleic acid molecule according to the present invention may comprise two or more labels, whereby the labels are preferably different from each other.

Particularly preferred fluorescent labels are FLUOS (Boehringer Mannheim, Mannheim, Germany), TRITC (Isomer G. Molecular Probes Inc.), CT (Molecular Probes Inc., Eugene in USA), CY-3 (Biological Detection Systems, Pittsburgh, USA), CY-5 (Biological Detection Systems, Pittsburgh, USA), FITC and CY 2.

Preferred radioactive labels are, among others, ³⁵S, ³²P, ³³P, and ¹²⁵J.

Preferred enzymatically active groups or molecules which may act as a label are preferably selected from the group comprising alkaline phosphatase, acid phosphatase, peroxidase, horseradish peroxidase, beta-D-galactosidase and glucose oxidase. For each of these enzymes, a number of chromogens is known which can be used instead of the natural substrate and which can be transformed into coloured or fluorescent products. Examples of such chromogenes are given in the following table:

| Enzymes | | Chromogen |
|---|---|---|
| 1. | Alkaline phosphatase and acid phosphatase | 4-methylumbelliferyl phosphate (*), bis(4-methylumbelliferyl phosphate), (*) 3-O-methylfluorescein, flavone-3-diphosphate triammonium salt (*), p-nitrophenylphosphate disodium salt. |
| 2. | Peroxidase | tyramine hydrochloride (*), 3-(p-hydroxyphenyl)-propionic acid (*), p-hydroxyphenethyl alcohol (*), 2,2'-azino-di-3-ethylbenzthiazoline sulfonic acid (ABTS), ortho-phenylendiamine dihydrochloride, o-dianisidine, 5-aminosalicylic acid, p-ucresol (*), 3,3'-dimethyloxy benzidine, 3-methyl-2-benzothiazoline hydrazone, tetramethylbenzidine |
| 3. | Horseradish peroxidase | H₂O₂ + diammonium benzidine H₂O₂ + tetramethylbenzidine |
| 4. | β-D-galactosidase | o-nitrophenyl-β-D-galactopyranoside, 4-methylumbelliferyl-β-D-galactoside |
| 5. | Glucose oxidase | ABTS, glucose and thiazolyl blue. |

| | | |
|---|---|---|
| * fluorescence | | |

Finally it is possible to design the nucleic acid probe molecules according to the invention in such a way that they have another nucleic acid sequence at their 5' or 3' end that is suitable for hybridization. This nucleic acid sequence again comprises approx. 12 to 1000, preferably 15 - 50 nucleotides. This second nucleic acid part can typically be recognized by an oligonucleotide probe detectable by any of the above mentioned compounds or agents.

Another possibility is the coupling of the detectable nucleic acid probe molecules with a hapten. After detaching the nucleic acid probe molecules from the target nucleic acid, the nucleic acid probe molecules, which are now present separately, can be contacted with detectable antibodies recognizing the hapten. A well known example of such a hapten is digoxigenin or its derivatives. The person skilled in the art knows many other possibilities apart from the here mentioned examples to detect and to quantify an oligonucleotide used for hybridization.

The multitude of possible labels further allows the simultaneous detection of the two of the groups of bacteria, i. e. the *E*. *coli* genogroup and the group of coliforms thus obviating the need to prepare a replica of the sample or take another aliquot of the sample to be analyzed. Thus, for example by using two or more different fluorescence markers, both groups can be detected simultaneously and differentiated.

The interpretation of test results depends on the kind of label used at the probe or primer. Within the scope of the present invention, the interpretation can be performed advantageously by a light-optical microscope, epifluorescence microscope, chemiluminometer, fluorometer and the like which will be acknowledged by the ones skilled in the art.

It is within the present invention that the various embodiments of the nucleic acid molecules according to the invention may be used individually or in any combination in each and any of the aspects of the present invention disclosed.

It is within the present invention that the nucleic acid molecules according to the present invention can be either used as a primer or as a probe.

In the embodiments of the various aspects of the present invention where the nucleic acid molecules according to present invention are used as primers, they are preferably used to amplify the target nucleic acid, i.e. the particular section of the *oriC* locus to which they bind, preferably by base-pairing, and adjacent stretches or regions, preferably at the 3'-end of the primer. Such amplification of the target of the nucleic acid molecule according to the present invention, i. e. particularly the region of the *oriC* locus specific for bacteria of the *E*. *coli* genogroup and bacteria of the group of coliforms, respectively, specifically amplifies the nucleic acid of said two groups of bacteria which in turn allows the detection of such target nucleic acid and ultimately of the bacteria containing such target nucleic acid. By such amplification, even very low numbers of target nucleic acid and thus of corresponding bacteria can be detected. Methods for amplification which use such primers are well known in the art and comprise, among others, polymerase chain reaction, ligase chain reaction, nucleic acid sequence-based amplification, self-sustained sequence replication, strand displacement amplification, branched DNA signal amplification, nested PCR, and multiplex PCR. Each and any of these methods are known to the one skill in the art (for example: Fred M. Ausubel (ed.) et al.(1987) Current protocols in molecular biology, Wiley & Sons, Washington). When used as a primer, the nucleic acid molecule according to the invention can comprise any label known to the ones skilled in the art including, but not limited to, those described herein, whereby it is nevertheless preferred that the primer does not have a bulky label. Most preferably, if any, the label is a fluorescent or radioactive label.

The nucleic acid molecules according to the present invention may also be used as probes. Such probes are, due to their specificity, suitable to specifically interact with the target nucleic acid, i.e. the *oriC* of the various bacteria of the E. *coli* genogroup and bacteria of the group of coliforms, respectively. As the presence of the target nucleic acid also depends on the presence of corresponding microorganisms, not only the nucleic acid as such but also, at least indirectly, the presence of a bacterium containing such nucleic acid can be assayed. As the probe is used for the detection of corresponding nucleic acid molecules, it preferably comprises a label known to the ones skilled in the art including, but not limited to, those described herein. Such label allows the detection of the target nucleic acid by means of the nucleic acid molecule according to the present invention which specifically interacts with the target nucleic acid and thus link the target nucleic acid molecule to the labeled nucleic acid molecule according to the present invention. Methods to ultimately detect the complex comprising a labeled nucleic acid molecule according to the present invention or the nucleic acid molecule according to the present invention, are known to the ones skilled in the art. Preferably, such detection methods involves separation of the nucleic acid molecule complex consisting of the nucleic acid molecule according to the present invention and the target nucleic acid molecule by means of electrophoresis or chromatography. If the running characteristics of the respective complex in such detection system(s) are known, it is also possible that the nucleic acid molecule according to the present invention which is used as a probe, is not labeled. It is also within the present invention that the nucleic acid molecule which is detected, is amplified in prior steps using the nucleic acid molecules according to the present invention as (a) primer(s). It is also within the present invention that the nucleic acid molecules according to the present invention are either simultaneously or at different points in time used as primer(s) and probe(s), respectively, in the various applications of detecting the bacteria for which they are specific. A particularly preferred method where the nucleic acid molecules according to the present invention are used as primer(s) and/or probe(s) is the polymerase chain reaction. It will be acknowledged by the ones skilled in the art that depending on the reaction systems used such as fast PCR, the reaction conditions and in particular the periods of time have to be adjusted accordingly to reach the required specificity. Reaction conditions for standard PCR reactions in terms of temperature are as follows: annealing temperatures range form 54 to 62°C, denaturing temperature is 95°C and extension temperature is 72°C.

In a further aspect, the present invention is related to a combination of the nucleic acid molecules according to the present invention. Such combination comprises at least one first nucleic acid molecule and at least one second nucleic acid molecule. The first nucleic acid molecule is a nucleic acid molecule according to the invention which specifically base-pairs with a part or a fragment of the oriC locus of bacteria of the group of coliforms, and the second nucleic acid molecule is a nucleic acid molecule according to the invention which specifically base-pairs with a part or a fragment of the *oriC* locus of bacteria of the *E*. *coli* genogroup. In a further embodiment the first nucleic acid molecule is a nucleic acid molecule having or comprising a nucleic acid sequence according to SEQ.ID.No. 1, SEQ.ID.No. 2 or SEQ.ID.No. 3. In a further embodiment the second nucleic acid molecule is a nucleic acid molecule having or comprising a nucleic acid sequence according to SEQ.ID.No. 4 or 5.

It is also within the present invention that, particularly when used as primers for amplifying the target nucleic acid the first nucleic acid molecule is actually a combination of a nucleic acid molecule comprising a nucleic acid sequence according to SEQ.ID.No. 1 and 2 and a combination of a nucleic acid molecule comprising the nucleic acid sequence according to SEQ.ID.No. 2 and 3. Furthermore, it is within the present invention that, particularly, when used as primers for amplifying the target nucleic acid the second nucleic acid molecule is actually a combination of a nucleic acid molecule comprising a nucleic acid sequence according to SEQ.ID.No. 4 and 5. In connection therewith, and mostly for reason of clarity, the term combination in this embodiment does not refer to two nucleic acid molecules having the respective nucleic acid sequences and which are covalently linked to each other, but the respective two nucleic acid molecules are two separate strands which are preferably used together in a method according to the present invention as individual nucleic acid molecules and as individual probes and primers, respectively.

In a further aspect, the present invention is related to a method of using probes or primers or both for determining the presence or amount of a nucleic acid, making use of the nucleic acid molecules according to the present invention. In a preferred embodiment, the nucleic acid is from bacteria of the group of coliforms or from bacteria of the *E*. *coli* genogroup with the specificities of these two groups being as disclosed herein. It will be acknowledged, that the nucleic acid is an indication or finger-print of the corresponding organism which, under natural conditions, contains such nucleic acid. In so far, the detection or determination of the presence of the nucleic acid allows a qualitative and/or quantitative assessment also of the presence of the respective organism(s) which is particularly useful in the field of medical diagnosis and food control, particularly water quality control.

As is known to the ones skilled in the art, such method does not only allow for a qualitative analysis of a sample, but also for a quantitative analysis. Quantitative analysis is possible due to the relationship between the signal provided by the label attached to the nucleic acid molecules according to the present invention and the amount of target nucleic acid molecules contained in the respective sample. It will be appreciated by the ones skilled in the art that as nucleic acid molecules in general are rather fragile molecules which are subject to both chemical as well as biological degradation, the presence of respective nucleic acid molecules is typically linked to an organism which either is or, prior to the treatment of the sample, was alive. Also in case the respective methods according to the present invention are applied to quantitative analysis involving the amplification of a target nucleic acid, such amplification can be controlled and allows the one skilled in the art to analyze the sample quantitatively with regard to the presence of bacteria of the *E*. *coli* genogroup and bacteria of the group of coliforms, respectively.

In a further aspect, the present invention is related to a method for detecting and/or identifying and/or quantifying bacteria of the group of coliforms in a sample. For such purpose, the nucleic acid molecules according to the present invention which are specific for the *oriC* locus of the group of coliforms, are used for detection purposes. In a still further aspect, the present invention is related to a method for the detection and/or identification and/or quantification of bacteria of the *E. coli* genogroup. For such purpose, the nucleic acid molecules according to the present invention which are specific for the *oriC* locus of the group of coliforms, are used for detection purposes. It will be acknowledged by the ones skilled in the art that, again, quantification is possible using the methods as disclosed herein.

Detection as preferably used herein, means giving experimental evidence or providing physical or chemical evidence that an organism, or a representative part of it, is present using one or a number of nucleic acid molecules according to the present invention. As preferably used herein, the term identification means the detection of the particular organism as defined above and assigning the organism to a particular group of organisms and bacteria in particular.

In a further aspect, the present invention is related to a method for differential detection and/or differential identification and/or differential quantification of bacteria of the group of coliforms and bacteria of the *E. coli* genogroup in a sample. With regard to the concept of indicator organisms and the need for differential analysis in the fields of medicine, hygiene and food safety, it is sometimes necessary and generally helpful in terms of time and efforts to be undertaken to first determine the absence or presence of bacteria of the group of coliforms. Once such analysis has been performed, there might be the need to differentiate the group of organisms actually detected. Using the methods according to the prior art, which typically involve a cultivation step, such differential diagnosis needed either a further sample or an aliquot from the first sample, or a replica of the respective colonies which were commonly needed anyway in order to provide a significant species- or group-specific signal, typically in terms of metabolic activity. Using the approach as disclosed herein, the same sample can be tested for both whether the target nucleic acid contained in the sample is belonging to the group of coliforms and more specifically whether the sample contained bacteria of the group of the *E. coli* genogroup. Due to the different regions on the respective strands to which the respective nucleic acid molecule(s) according to the present invention bind, which is/are either used as primers or as probes, a specific detection is possible, particularly if the label of the different nucleic acid molecules according to the present invention which are used to specifically detect the coliforms and E. *coli* genogroup bacteria, respectively, differ from each other. In a particularly preferred embodiment, the first nucleic acid molecule according to the present invention, i.e. preferably a nucleic acid molecule having a nucleic acid sequence according to SEQ.ID.No. 1, 2 or 3, has a first label, and the second nucleic acid molecule according to the present invention, i.e. preferably a nucleic acid molecule having a nucleic acid sequence according to SEQ.ID.No. 4 or 5, has a second label, whereby the first label is different from the second label and most preferably the labels are fluorescent labels. Similar to the individual steps of detecting either bacteria of the group of coliforms or detecting bacteria of the *E. coli* genogroup, the same procedural steps as defined above are applicable for this kind of differential detection, differential identification or differential quantification.

In connection with the various methods according to the present invention, in most cases it is necessary to prepare the nucleic acid from the bacteria to be detected, identified or quantified in view of the mechanism underlying such methods. Although it is possible that, at least to a certain extent, nucleic acid molecules or fragments may be released from spontaneously dying cells, such release is not a proper reflection of the overall content of the bacteria to be detected. Further, it is usually necessary or recommendable for a reliable analysis to recover more or less all of the undegraded target DNA sequences from the cells. Such recovery requires, in a first step, cell lysis. Cell lysis may be effected by various means known to the ones skilled in the art, including cell lysis by chemical or physical agents. Preferably, the recovery of the target nucleic acid by microbial lysis may be effected by brief exposure to extremes of pH, temperatures such as by boiling or freezing/thawing, sonification, microwaves, organic solvents, chaotropic agents like urea, guanidium thiocyanate or guanidine HCl, detergents like sodium dodecyl sulfate (SDS) and Triton X-100, osmotic shock, lysozyme digestion or protease digestion and the like. Optionally interfering substances can be removed, for example, by organic solvent extraction, acid precipitation, ultrafiltration, solid-phase extraction, HPLC, LiCl precipitation, protease digestion, RNase digestion or polyethylenglycol precipitation and the like. Solid-phase extraction or HPLC can be based on ion-exchange, reverse-phase, hydrophobic-interaction or silicagel adsorption interactions. Preferably, release of DNA from target organisms, i. e. bacteria of the *E. coli* genogroup and bacteria of the group of coliforms, is performed by the use of SDS-lysozyme treatment, specifically alternation of freeze (-70°C) thaw (25°C) cycles or boiling.

A further step which might be involved in any of the methods according to the present invention is to recover the target cells from the sample. Whether or not such recovery is necessary, strongly depends on the sample. For example, if the sample as such already is highly infected by bacteria or allegedly highly infected by the target organism(s), a further recovery or enrichment might not be necessary. Rather it could be within the present invention to directly use some of the bacteria obviously forming or being present on the sample, typically as colonies or biofilms. Nevertheless, further recovery or purification prior to submitting the bacteria to any procedures allowing access to the undegraded DNA contained in the target organisms, is comprised by the present invention.

If the sample is highly diluted and there are reasons to assume that the concentration of target organisms is not very high, the target organisms can be recovered in preferred embodiments of the various methods of the present invention. Preferably such recovery is necessary in case of assessing the quality of water, in particular drinking water, where the number of coliforms has to be very low in accordance with hygiene standards of most countries. Typically, a water sample is taken and subjected to several suitable means, including, for example, filtration centrifugation, possibly with the help of suspended or dissolved additives which serve to capture or flocculate the target organisms in a physical state which facilitates their separation. If the microorganisms are not adsorbed to much larger particles or flocculated, the normal filter pore size should be no larger than about 0.2-0.5 µm, preferably about 0.45 µm, to assure efficient capture. If the microorganisms are recovered in a gel or adsorbed to particles, much larger filter pore size are preferred to accelerate filtration. Preferred cell recovery according to this invention is obtained by centrifugation from small volume samples, about 1 ml or less, and by filtration for small or large, typically 100 ml, volume samples. Especially preferred cell recovery by filtration of this invention is by passage through 11-13 mm diameter 0.20-0.50 µm pore size polycarbonate or Teflon filters.

In a further aspect, the present invention is related to a solid support comprising at least one or several of the nucleic acid molecules according to the present invention or a combination according to the present invention thereof. Preferably, the respective nucleic acid molecules are attached to a solid support. Such solid support is preferably selected from the group comprising glass, cellulose and metals, or from supports comprising a surface consisting of such material. The nucleic acid molecules according to the present invention may be either covalently or non-covalently attached to said surface. The respective solid support, which as such, is also known in the art as biochip, is used in a preferred embodiment in any of the methods according to the present invention. The detailed steps on how to use such biochips are known to the one skill in the art and are, for example, described in Bryant et al. (2004) Chips with everything: DNA microarrays in infectious diseases. Lancet Infect. Dis. 4, 100-11.

In a further aspect the present invention is related to kits. Such kits may actually be used in any method according to the present invention. The kit comprises at least one nucleic acid according to the present invention. The specificity of the respective nucleic acid molecule defines the bacteria or group of bacteria which may be detected, identified or quantified when using the respective kit. Apart from the specific nucleic acid molecule(s), such kit may optionally comprise one or several of the following components: positive controls, negative controls, buffers, surfaces and detection means, as defined herein. In a preferred embodiment, the respective chemical compounds for performing the methods according to the present invention using the kit according to the present invention are packaged or are present as aliquots such as to allow a single or multitude of reactions.

The present invention will now further be illustrated by the attached examples, figures and the sequence protocol from which further features, embodiments and advantages of the present invention can be taken.

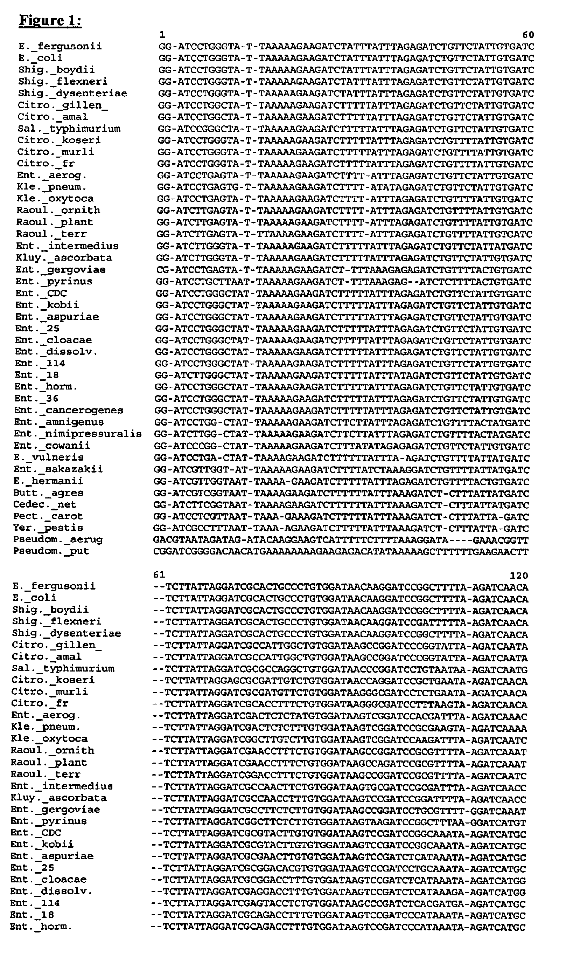

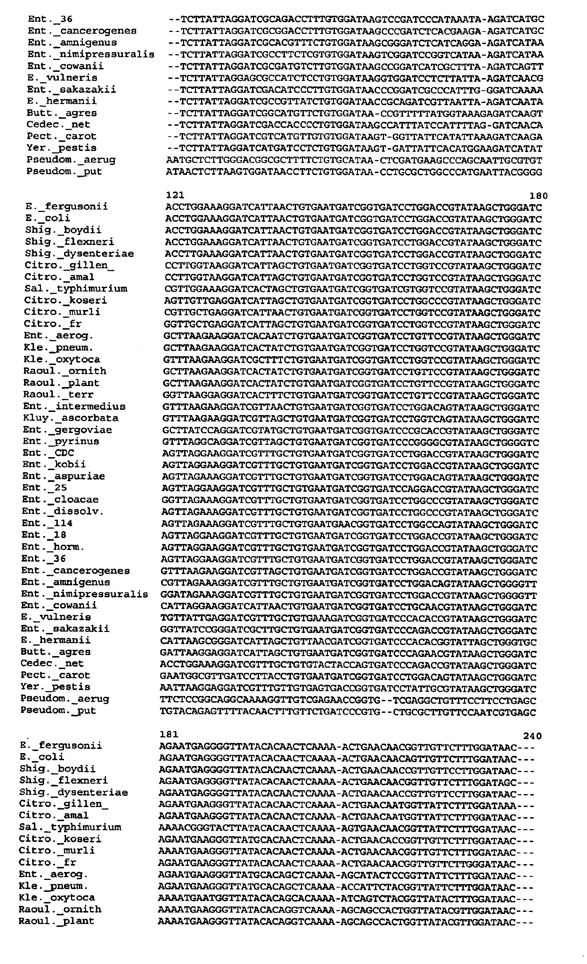

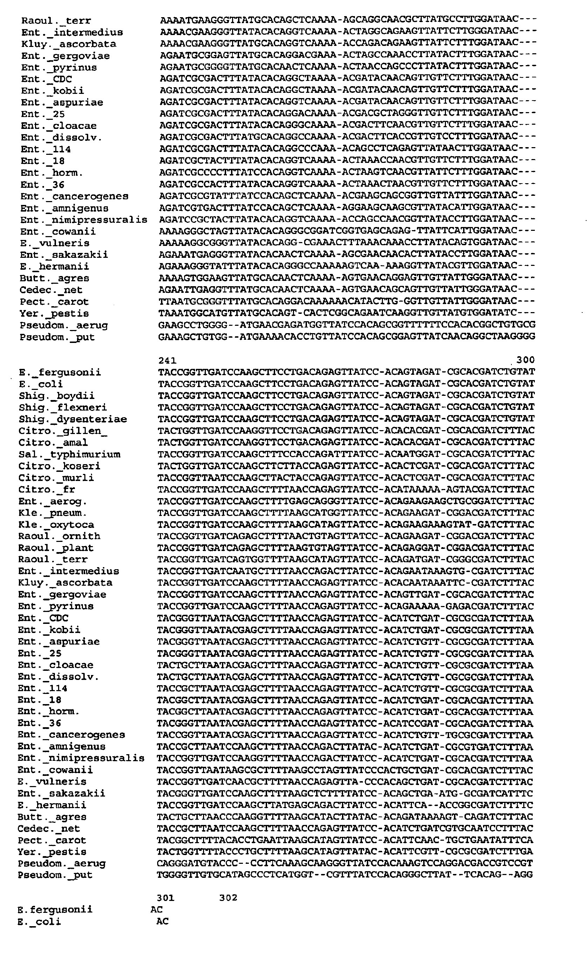

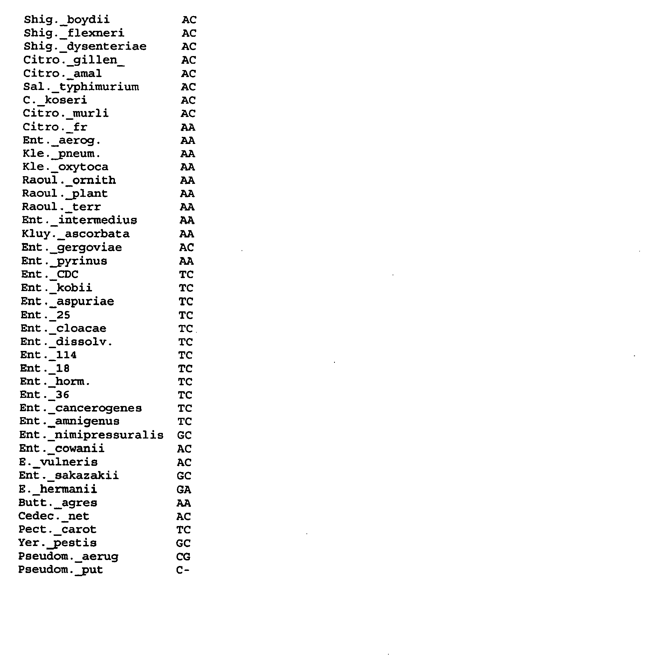

Fig. 1 shows the result of a phylogenetic analysis depicted as a neighbor-joining tree

### Example 1: Material and methods

Unless indicated to the contrary, the following examples were carried out using the methods and materials described herein.

**Sample preparation**. Reference strains were grown in LB at 37 °C to an OD₆₀₀ of 1.0, diluted 1:100 in sterile, distilled water and subjected to 10 min. boiling. Water samples or solutions with or without bacteria were concentrated by filtration using a 0.45 µm pore size polycarbonate filter (Millipore GmbH, Schwalbach, Germany) or by centrifugation at 5,000 g for 10 min. Bacterial Pellets were washed in 1ml sterile, distilled water, re-suspended in 10 - 100 µl of sterile, distilled water and boiled for 10 min. in order to release DNA.

**PCR assays.** PCR primers were designed based on conserved regions of the alignment of *oriC* loci of different Enterobactereaceae. The sequences of the following GenBank accession numbers were used for comparison or for the design of PCR primers: *Escherichia coli* AE000451, *Enterobacter aerogenes* J01576, *Klebsiella pneumoniae* J01744.1, *Pectobacterium carotovorum* NC004547, *Pseudomonas aeruginosa* M30125, *Pseudomonas putida* M30126, *Salmonella enterica* Serovar Typhimurium NC003197, *Salmonella enterica* Serovar Typhi AL627280 *Salmonella enterica* Serovar Enteritidis AED16846, *Shigella flexneri* X67659, *Shigella boydii* X67658, *Shigella dysenteriae* X67657, *Shigella sonneii* X67655, *Yersinia pestis* NC004088, *Yersinia pseudotuberculosis* NC006155, *Photorhabdus luminescens* BX571859, *Vibrio cholera* AY211526.1, *Vibrio vulnificus* AE016800.1, *Shewanella oneidensis* AE014299, *Pasteurella multocida* AE004439. PCR assays were performed using a Perkin-Elmer Gene Amp PCR System 2400 or an Applied BioSystems GeneAmp PCR System 2700 and *Taq* Gold DNA polymerase (Perkin-Elmer, Branchburg, N. J.). Thin walled PCR tubes were purchased from Applied Biosystems (Branchburg, N.J.). Primers were synthesised by Metabion, Munich, Germany. Reactions were performed in a total volume of 50 µl under standard conditions (500 pmol of each primer, 200 µM nucleotide mix, 10 mM Tris-Cl (pH 8,3), 5 mM KCI, 1.5 mM MgCl₂ and 2.5 U of *Taq* polymerase) with 5 µl sample preparation. Reactions were started after a preheat time of 10 min at 95°C. Samples were subjected to 35 cycles of 30 s at 95°C, 30 s at annealing temperature and 1:30 min extension at 72°C. Cycles were followed by a final elongation step of 15 min at 72°C. Annealing temperatures (AT) were optimized by step-wise increasing the AT by 2°C starting at 6°C below the lowest Tₘ of the primer sets used, until specific, single banded PCR products were obtained. The following annealing temperatures were chosen: 56°C for PCRs using primer SEQ.ID.No.1, 2 and 3, and 58°C for PCR using SEQ.ID.No.4 and 5. PCR products were visualised in 2.0% agarose gels after electrophoresis and ethidium bromide staining.

**DNA sequencing.** PCR-amplified DNA was sequenced using the dye terminator method in both directions. To prepare cycle sequencing reactions, 0.2 µg of purified DNA was added to a 20 µl reaction solution containing 4 µl of BigDye® dye terminator sequencing mix and 0.7 µl of a 5 pmol primer solution. Cycle sequencing was performed in the Applied BioSystems GeneAmp PCR System 2700. Cycle parameters were 25 cycles with an initial 96°C denaturation step of 10 s, followed by an annealing step at 57°C of 10 s and an extension step of 4 min at 60°C. Sequences were determined by electrophoresis with the ABI PRISM 377 DNA sequencer. About 1‰ of bp sequenced resulted in an "N". These have been corrected based on the respective reverse sequences using the SeqMan (DNAStar) program.

**Data analysis.** Sequence data were analysed using MegAlign (DNAStar Inc., 1999) and PAUP version 4.0b10 (Swofford, 1998) on a Macintosh G4 PC. Multiple alignment was performed using Clustal V included in the MegAlign program. Using MegAlign, pairwise percent sequence divergence was calculated as 100 times the sum of the residue distances from one strain to the other along the tree divided by the sum of all branch lengths. Using PAUP 4.0b10, Neighbour-Joining trees (Saitu and Nei, 1987) were estimated based on pair wise genetic distances on the basis of all substitutions using the Jukes-Cantor distance parameter. The significance of branchings was evaluated by bootstrap analysis of 100 replicates. Only groups with frequencies above 50% were kept. Bootstrap values above 80% were considered significant and the respective cluster was designated "strongly supported".

### Example 2: Specification of bacteria of the E.coli genogroup and bacteria of the group of coliforms

Using the methods described in example 1, a combination of two nucleic acid molecules having a nucleic acid sequence according to SEQ.ID.No. 4 and 5 (PCR 1 primer set), SEQ.ID.No: 1 and 3 (PCR 2 primer set),or SEQ.ID.No. 2 and 3 (PCR 3 primer set) was used to test a number of reference strains of bacteria of the E. *coli* genogroup and of the group of coliforms.

The results are presented in the following table.

| *Species* | strain | PCR 1^{a} | PCR 2^{b} | PCR 3^{c} |
|---|---|---|---|---|
| *Escherichia coli* | K12, DH5α | + | + | + |
| *Escherichia coli* | ATCC 25922 | + | + | + |
| *Escherichia coli* | EHEC 1392 | + | + | + |
| *Escherichia fergusonii* | DSM 13698 | + | + | + |
| *Escherichia hermanii* | DSM 4560 | - | + | + |
| *Escherichia vulneris* | DSM 4564 | - | + | + |
| *Shigella flexneri* | MvP^{d} | + | + | + |
| *Salmonella typhimurium* | MvP | - | + | + |
| *Kluyvera ascorbata* | DSM 4611 | - | + | + |
| *Klebsiella pneumoniae* | DSM 30104 | - | + | + |
| *Klebsiella oxytoca* | DSM5175 | - | + | + |
| *Enterobacter aerogenes* | DSM 30053 | - | + | + |
| *Raoultella ornithinolytica* | DSM 7464 | - | + | + |
| *Raoultella planticola* | DSM 3069 | - | + | + |
| *Raoultella terrigena* | DSM 2687 | - | + | + |
| *Enterobacter cloacae* | ATCC 13047 | - | + | + |
| *Enterobacter dissolvens* | ATCC 23373 | - | + | + |
| *Enterobacter kobei-*II | CDC 1347-71 | - | + | + |
| *Enterobacter kobei* | DSM 13645 | - | + | + |
| *Enterobacter hormaechei* | ATCC 49162 | - | + | + |
| *Enterobacter nimipressuralis* | ATCC 9912 | - | + | + |
| *Enterobacter aspuriae* | ATCC 35953 | - | + | + |
| *Enterobacter cancerogenes* | ATCC 33241 | - | + | + |
| *Enterobacter intermedius* | ATCC 33110 | - | + | + |
| *Enterobacter amnigenus* | ATCC 3072 | - | + | + |
| *Enterobacter sakazakii* | ATCC 29544 | - | + | + |
| *Enterobacter gergoviae* | ATCC 33028 | - | + | + |
| *Enterobacter pyrinus* | ATCC 49851 | - | + | + |
| *Enterobacter cowenii* | CIP 107300 | - | + | + |
| *Enterobacter sp.* | 18 | - | + | + |
| *Enterobacter sp.* | 25 | - | + | + |
| *Enterobacter sp.* | 36 | - | + | + |
| *Enterobacter sp.* | 114 | - | + | + |
| *Citrobacter freundii* | DSM 30039 | - | + | + |
| *Citrobacter koseri* | DSM 4595 | - | + | + |
| *Citrobacter amalonaticus* | DSM 4593 | - | + | + |
| *Citrobacter gillenii* | DSM 13694 | - | + | + |
| *Citrobacter murliniae* | DSM 13695 | - | + | + |
| *Pectobacterium carotovorum* | DSM 30168 | - | + | + |
| *Buttiauxella agrestis* | DSM4586 | - | + | + |
| *Hafnia alvei* | DSM 30163 | - | - | - |
| *Pantoea agglomerans* (basonym: *Erwinia herbicola)* | DSM 3493 | - | - | - |
| *Pantoea terrae* | DSM 13701 | - | - | - |
| *Pantoea ananatis* (basonym: *Erwinia ananatis)* | DSM 30080 | - | - | - |
| *Leclercia adecarboxylata* | DSM 5077 | - | + | - |
| *Serratia marcescens* | ATCC 9141 | - | + | - |
| *Serratia liquefaciens* | DSM 4487 | - | - | - |
| *Serratia fonticola* | DSM 4576 | - | - | - |
| *Rahnella aquaticus* | DSM4594 | - | - | - |
| *Proteus vulgaris* | ATCC 13315 | - | - | - |
| *Proteus mirabilis* | ATCC 43071 | - | - | - |
| *Morganella morganii* | DSM 6675 | - | - | - |
| *Aeromonas hydrophilia* | MvP | - | - | - |
| *Plesiomonas shigelloides* | MvP | - | - | - |
| *Pseudomonas aeruginosa* | ATCC 27853 | - | - | - |
| *Legionella pneumophilia* | ATCC 33152 | - | - | - |

| | | | | |
|---|---|---|---|---|
| ^{a}PCR1 primer set: SEQ.ID.No. 4 and SEQ.ID.No. 5 ^{b}PCR2 primer set: SEQ.ID.No. 1 and SEQ.ID.No. 3 ^{c} PCR3 primer set: SEQ.ID.No. 2 and SEQ.ID.No. 3 ^{d} MvP: laboratory strain of the Max von Pettenkofer-institute | | | | |

As may be taken from the table, the PCR 3 primer set and PCR 2 primer set are suitable to detect a number of organisms all of which are generally regarded as belonging to the group of coliforms. In so far, the particular nucleic acid molecules and the combinations thereof are suitable for the detection of each and any of the microorganisms specified in the above table where the respective strain is characterized as being positive ("+").

Using the combination of a nucleic acid molecule having a nucleic acid sequence according to SEQ.ID.No. 4 and a nucleic acid molecule having a nucleic acid sequence according to SEQ.ID.No. 5, a specific detection of the *E. coli* genogroup organisms is obtained which are represented by the corresponding reference type strains in the above table.

From these results it is evident that a sample which is subject to an analysis for the presence or absence of bacteria of the group of coliforms using the PCR 2 primer set and the PCR primer set 3, respectively, can subsequently be subjected to an analysis for the presence of specific members of the group of coliforms, namely the bacteria generally referred to as bacteria of the *E. coli* genogroup by using primer SEQ.ID.No. 4 or SEQ.ID.No. 5 or a combination of both.

### Example 3: Phylogenetic relationship between bacteria of the group of coliforms

Based on the finding underlying the present invention, the present inventor has sequenced the core region of the *oriC* locus comprising about 290 base pairs of various species of the *Enterobacteriaceae* family and compared it to the corresponding gene locus of *Yersinia pestis* (NC 004088) and *Pseudomonas* spp (ACC.No. M30125, M30126). The result of such analysis is depicted in figure 1, whereby the abbreviations used are as follows:
E.: Escherichia; Shig.: Shigella; Citro.: Citrobacter, Sal.: Salmonella; Ent.: Enterobacter, Kle.: Klebsiella; Kluy.: Kluyvera; Raoul.: Raoultella; Butt.: Buttiauxella; Cedec.: Cedecea; Pect.: Pectobacterium; Yer.: Yersinia, Pseudom.: Pseudomonas.

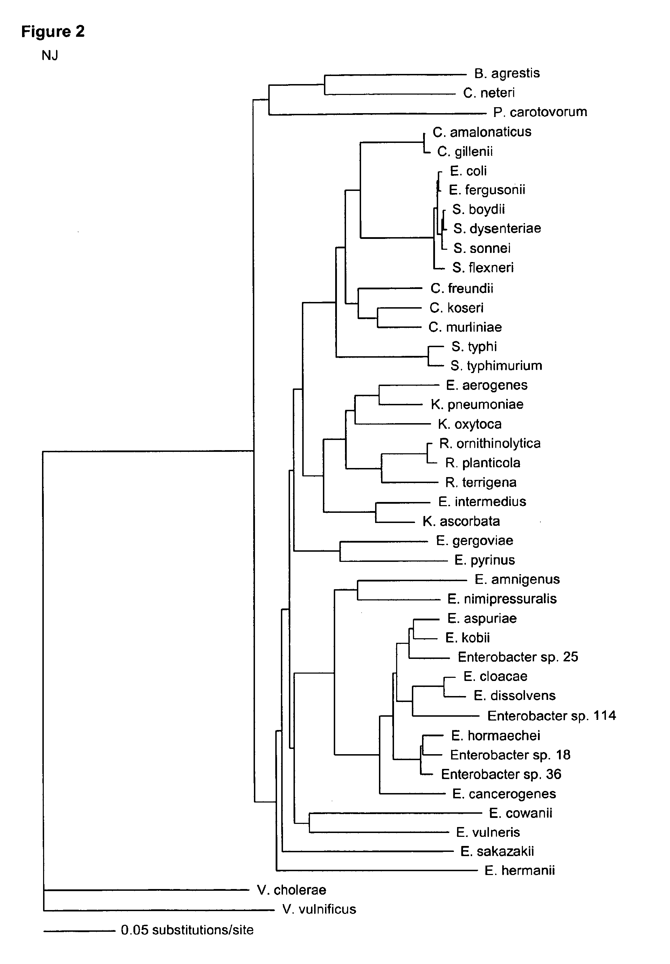

Using this information a neighbour-joining tree using the PAUP software packet based on the central 290 bp of the *oriC* locus of various *Enterobacteriaceae* were generated. The result thereof is depicted in Fig. 2.

The features of the present invention disclosed in the specification, the claims and/or the drawings may both separately and in any combination thereof be material for realizing the invention in various forms thereof.

## Claims

1. A combination of at least a first nucleic acid molecule and at least a second nucleic acid molecule for the detection of and/or discrimination between bacteria of the group of coliforms and bacteria of the *Escherichia coli* genogroup, whereby the first nucleic acid and the second nucleic acid differ in their nucleic acid sequence and comprise fragments of the origin of replication *(oriC)* locus sequence of said bacteria.

2. The combination according to claim 1, whereby the first and the second nucleic acid molecule each and independently have a length of at least 14 or 15 nucleotides.

3. The combination according to claim 1 or 2, whereby the first and the second nucleic acid molecule each and independently have a maximum length of 600 nucleotides, preferably 300, more preferably 50 and most preferably 30 nucleotides.

4. A combination of at least a first nucleic acid molecule and a second nucleic acid molecule, preferably according to any of claims 1 to 3, whereby the first nucleic acid molecule is selected from the group comprising
a nucleic acid molecule comprising the following nucleic acid sequence:
5'-CAAAAGGATCCKGATAAAACATGGT-3' (SEQ. ID. No. 1),
a nucleic acid molecule comprising the following nucleic acid sequence:
5'-CGGGCCGTGGATTCTACTCA-3' (SEQ. ID. No. 2), and
a nucleic acid molecule comprising the following nucleic acid sequence:
5'-CATGAGATTCCGGARGATCCG-3' (SEQ. ID. No. 3); and
whereby the second nucleic acid molecule is selected from the group comprising
a nucleic acid molecule comprising the following nucleic acid sequence:
5'-CACTGCCCTGTGGATAACAA-3' (SEQ. ID. No.4), and
a nucleic acid molecule comprising the following nucleic acid sequence:
5'-TATACAGATCGTGCGATCTAC-3' (SEQ. ID. No. 5).

5. The combination according to claim 4, whereby the first nucleic acid molecule is a combination of a nucleic acid molecule comprising a nucleic acid sequence according to SEQ.ID.No. 1 and a nucleic acid molecule comprising a nucleic acid sequence according to SEQ.ID.No. 3, or a combination of a nucleic acid molecule comprising a nucleic acid sequence according to SEQ.ID.No. 2 and a nucleic acid molecule comprising a nucleic acid sequence according to SEQ.ID.No. 3.

6. The combination according to any of claims 4 to 5, whereby the second nucleic acid molecule is a combination of a nucleic acid molecule comprising a nucleic acid sequence according to SEQ.ID.No. 4 and a nucleic acid molecule comprising a nucleic acid sequence according to SEQ.ID.No. 5

7. A nucleic acid molecule, whereby the nucleic acid molecule is selected from the group comprising
a nucleic acid molecule comprising the following nucleic acid sequence:
5'-CAAAAGGATCCKGATAAAACATGGT-3' (SEQ. ID. No.1),
a nucleic acid molecule comprising the following nucleic acid sequence:
5'-CGGGCCGTGGATTCTACTCA-3' (SEQ. ID. No. 2), and
a nucleic acid molecule comprising the following nucleic acid sequence:
5'-CATGAGATTCCGGARGATCCG-3' (SEQ. ID. No. 3).

8. A nucleic acid molecule, whereby the nucleic acid molecule is selected from the group comprising
a nucleic acid molecule comprising the following nucleic acid sequence:
5'-CACTGCCCTGTGGATAACAA-3' (SEQ. ID. No.4), and
a nucleic acid molecule comprising the following nucleic acid sequence:
5'-TATACAGATCGTGCGATCTAC-3' (SEQ. ID. No. 5).

9. The nucleic acid molecule according to claim 7 or 8, whereby the nucleic acid molecule is used as a primer and/or as a probe.

10. A method using probes or amplification primers or both for determining the presence or amount of nucleic acid, whereby the nucleic acid is from bacteria of the group of coliforms or from bacteria of the *E*. *coli* genogroup, whereby the probes or amplification primers are derived from bacteria of the *oriC* locus of the group of coliforms or from bacteria of the *E*. *coli* genogroup.

11. The method according to claim 10, whereby the probes or amplification primers comprise a nucleic acid sequence according to SEQ.ID.No. 1, SEQ.ID.No. 2 or SEQ.ID.No. 3 and are preferably used for determining the presence or amount of bacteria of the group of coliforms or a nucleic acid thereof.

12. The method according to claim 10 or 11, whereby the probes or amplification primers comprise a nucleic acid sequence according to SEQ.ID.No. 4 or SEQ.ID.No. 5 and are preferably used for determining the presence or amount of bacteria of the E. *coli* genogroup or a nucleic acid thereof.

13. A method for the detection and/or identification and/or quantification of bacteria of the group of coliforms in a sample, comprising the step of
- detecting a nucleic acid fragment of the *oriC* locus of the group of coliforms.

14. The method according to claim 13, whereby in the step of detecting a nucleic acid fragment a nucleic acid molecule as defined in claim 7 is used.

15. A method for the detection and/or identification and/or quantification of bacteria of the *E. coli* genogroup, comprising the step of
- detecting a nucleic acid fragment of the *oriC* locus of bacteria of the E. *coli* genogroup.

16. The method according to claim 25, whereby in the step of detecting a nucleic acid molecule a nucleic acid molecule as defined in claim 8 is used.

17. A method for differential detection and/or differential identification and/or differential quantification of bacteria of the group of coliforms and of bacteria of the E. *coli* genogroup in a sample, comprising the steps of
a) providing a nucleic acid preparation of the sample, and
b) contacting a combination of at least a first nucleic acid molecule and a second nucleic acid molecule according to any of claims 1 to 6 with the nucleic acid preparation.

18. The method according to claim 17, whereby the method further comprises as step
c) detecting in the nucleic acid preparation a nucleic acid molecule which or a part of which is either essentially identical to or essentially complementary to the first and/or the second nucleic acid molecule.

19. The method according to claims 17 or 18, whereby the bacteria of the group of coliforms is detectable by the first nucleic acid molecule and the group of bacteria of the *E. coli* genotype is detectable by the second nucleic acid molecule.

20. The method according to any of claims 17 to 19, whereby the first and/or the second nucleic acid molecule is independently and individually used as primers and/or as probes.

21. A kit for the differential detection and/or differential identification and/or differential quantification of bacteria of the group of coliforms and of bacteria of the *E.*coli genogroup comprising at least one nucleic acid according to claim 7 and at least one nucleic acid according to claim 8.
